# EUROPEAN PATENT APPLICATION

(11) **EP 4 847 034 A1**
(43) Date of publication of application: **02.12.2026**
(21) Application number: 24920184.9
(22) Date of filing: 17.12.2024
(51) Int. Cl.: A61K 38/17, A61K 35/76, A61K 48/00, A61P 1/16, A61P 29/00, A61P 37/06, A61P 43/00, C07K 14/46, C12N 15/12, C12N 15/63, C12N 15/864

(54) **COMBINATION FOR TREATING AND/OR PREVENTING LIVER DISEASE**

(30) Priority: 24.01.2024 JP 2024008691
(71) Applicant: Kyushu University, National University Corporation, Nishi-ku Fukuoka-shi Fukuoka 819-0395 (JP)
(72) Inventor: SUZUKI Atsushi, Fukuoka-shi, Fukuoka 819-0395 (JP)
(74) Representative: J A Kemp LLP
(86) International application number: PCT/JP2024/044512
(87) International publication number: WO 2025/158828

(57) **Abstract**

In one embodiment, the present invention relates to a combination for treating and/or preventing liver disease, the combination comprising HNF1 gene or HNF1 protein, FOXA gene or FOXA protein, and HNF6 gene or HNF6 protein.

## Description

### Technical Field

In one embodiment, the present invention relates to a combination for treating and/or preventing liver disease.

### Background Art

There exists no effective method for treating liver cirrhosis, liver fibrosis, and NASH which are included in liver disease. For the treatment of severe liver disease, liver transplantation, which faces challenges in securing or matching donors, is the only option left if medical treatment reaches the limit. On the other hand, artificial liver under development worldwide cannot sufficiently substitute for functions of the liver and has not yet been in practical use.

### Summary of Invention

In one embodiment, an object of the present invention is to provide a drug or a method for treating and/or preventing liver disease.

The present inventor has found that liver disease can be treated and/or prevented by using a combination of specific reprogramming factors. The present invention encompasses the following embodiments.
(1) A combination for treating and/or preventing liver disease, the combination comprising HNF1 gene or HNF1 protein, FOXA gene or FOXA protein, and HNF6 gene or HNF6 protein.
(2) The combination according to (1), wherein the liver disease is selected from the group consisting of liver cirrhosis, liver fibrosis, and nonalcoholic steatohepatitis (NASH).
(3) The combination according to (1), wherein the liver disease is liver cirrhosis or liver fibrosis.
(4) The combination according to any of (1) to (3), wherein HNF1 is HNF1A.
(5) The combination according to any of (1) to (4), wherein FOXA is FOXA3.
(6) The combination according to any of (1) to (5), wherein the combination comprises the HNF1 gene, the FOXA gene, and the HNF6 gene.
(7) The combination according to (6), wherein the HNF1 gene, the FOXA gene, and the HNF6 gene are contained in a vector.
(8) The combination according to (7), wherein the HNF1 gene, the FOXA gene, and the HNF6 gene are contained in separate vectors.
(9) The combination according to (7) or (8), wherein the vector is adeno-associated virus (AAV).
(10) The combination according to (9), wherein the vector is AAV8.
(11) The combination according to any of (7) to (10), wherein the HNF1 gene, the FOXA gene, and the HNF6 gene are operably linked to hepatocyte-specific promoters in the vector.
(12) The combination according to any of (1) to (6), wherein the HNF1 gene, the FOXA gene, and the HNF6 gene are contained in lipid nanoparticles.
(13) The combination according to any of (1) to (12), wherein the combination is a pharmaceutical composition.
(14) A method for treating and/or preventing liver disease, comprising administering HNF1 gene or HNF1 protein, FOXA gene or FOXA protein, and HNF6 gene or HNF6 protein to a subject.
(15) The method according to (14), wherein the liver disease is selected from the group consisting of liver cirrhosis, liver fibrosis, and nonalcoholic steatohepatitis (NASH).
(16) The method according to (14), wherein the disease is liver cirrhosis or liver fibrosis.
(17) The method according to any of (14) to (16), wherein HNF1 is HNF1A.
(18) The method according to any of (14) to (17), wherein FOXA is FOXA3.
(19) The method according to any of (14) to (18), wherein the method comprises administering the HNF1 gene, the FOXA gene, and the HNF6 gene.
(20) The method according to (19), wherein the HNF1 gene, the FOXA gene, and the HNF6 gene are contained in a vector.
(21) The method according to (20), wherein the HNF1 gene, the FOXA gene, and the HNF6 gene are contained in separate vectors.
(22) The method according to (20) or (21), wherein the vector is adeno-associated virus (AAV).
(23) The method according to (22), wherein the vector is AAV8.
(24) The method according to any of (21) to (23), wherein the HNF1 gene, the FOXA gene, and the HNF6 gene are operably linked to hepatocyte-specific promoters in the vector.
(25) The method according to any of (14) to (19), wherein the HNF1 gene, the FOXA gene, and the HNF6 gene are contained in lipid nanoparticles.
(26) The method according to any of (14) to (25), wherein the HNF1 gene or the HNF1 protein, the FOXA gene or the FOXA protein, and the HNF6 gene or the HNF6 protein are contained in a pharmaceutical composition.

The present invention provides, for example, a drug or a method for treating and/or preventing liver disease.

### Brief Description of Drawings

[Figure 1] Figure 1A shows results of gene expression analysis on AFP and EpCAM when human iHepPC-inducing factors were introduced to a liver fibrosis mouse model using AAV8. Figure 1B shows results of EpCAM immunostaining when human iHepPC-inducing factors were introduced to a liver fibrosis model using AAV8.
[Figure 2] Figure 2 shows results of staining a fibrotic tissue (A), a relative value of the fibrotic tissue (B), a gene expression level of Col1a1 (C), and an amount of alanine aminotransferase (ALT) (D) and an amount of aspartate aminotransferase (AST) (E) in serum when human iHepPC-inducing factors were introduced to a liver fibrosis mouse model using AAV8.
[Figure 3] Figure 3 shows results of measuring fat deposition when human iHepPC-inducing factors were introduced to a liver fibrosis mouse model using AAV8.
[Figure 4] Figure 4 shows results of histopathological analysis when human iHepPC-inducing factors were introduced to a liver fibrosis mouse model using AAV8.
Figure 4A shows results of hematoxylin-eosin (HE) staining, and Figure 4B shows results of NAFLD activity score (NAS).
[Figure 5] Figure 5 shows results of staining a fibrotic tissue (A), a proportion of the fibrotic tissue (B), a gene expression level of Col1a1 (C), and an amount of alanine aminotransferase (ALT) (D) and an amount of aspartate aminotransferase (AST) (E) in serum when human iHepPC-inducing factors were introduced to a liver fibrosis mouse model using LNP.
[Figure 6] Figure 6 shows results of measuring fat deposition when human iHepPC-inducing factors were introduced to a liver fibrosis mouse model using LNP.
[Figure 7] Figure 7 shows results of histopathological analysis when human iHepPC-inducing factors were introduced to a liver fibrosis mouse model using LNP.
Figure 7A shows results of hematoxylin-eosin (HE) staining, and Figure 7B shows results of NAFLD activity score (NAS).

### Description of Embodiments

In one embodiment, the present invention relates to a combination for treating and/or preventing liver disease, the combination comprising HNF1 gene or HNF1 protein, FOXA gene or FOXA protein, and HNF6 gene or HNF6 protein (the "combination" is also referred to as a "combination product" in the present specification).

Examples of the liver disease as described herein include simple steatosis, nonalcoholic fatty liver disease (NAFLD) (also called "metabolic dysfunction-associated steatotic liver disease (MASLD)"), nonalcoholic steatohepatitis (NASH) (also called "metabolic dysfunction-associated steatohepatitis (MASH)"), alcoholic liver disease (ALD), alcohol-related liver disease, cholestatic liver disease, liver fibrosis, liver cirrhosis, liver failure, hepatitis, steatohepatitis, viral liver disease (e.g., hepatitis A, hepatitis B, and hepatitis C), hepatic ischemia, and liver cancer. Further examples of the liver disease include congenital liver disease (e.g., inborn errors of metabolism, congenital hepatic fibrosis, and Alagille syndrome), autoimmune liver disease, biliary atresia, acute liver failure, and drug-induced liver injury. The liver disease may be selected from the group consisting of, for example, liver cirrhosis, liver fibrosis, and nonalcoholic steatohepatitis (NASH), and can be, for example, liver cirrhosis or liver fibrosis.

As used herein, the "treatment" includes improvement of symptoms related to the disease as well as continuation of improved symptoms, suppression of recurrence, and all treatments related to the disease. As used herein, the "prevention" includes prevention of symptoms related to the disease or reduction in the risk thereof.

The combination described as described herein can be a combination of reprogramming factors. As used herein, the "reprogramming" means a process of changing the differentiated state of a cell to a differentiated state different from that of the cell or an undifferentiated state. Examples of factors for use in such reprogramming (reprogramming factors) include a combination comprising or consisting of HNF1 gene or HNF1 protein, FOXA gene or FOXA protein, and HNF6 gene or HNF6 protein. Specific examples of the combination include any of the following combinations 1) to 8), for example, a combination 1) or 8):
1) a combination of the HNF1 gene, the FOXA gene, and the HNF6 gene,
2) a combination of the HNF1 gene, the FOXA protein, and the HNF6 gene,
3) a combination of the HNF1 gene, the FOXA gene, and the HNF6 protein,
4) a combination of the HNF1 gene, the FOXA protein, and the HNF6 protein,
5) a combination of the HNF1 protein, the FOXA gene, and the HNF6 gene,
6) a combination of the HNF1 protein, the FOXA protein, and the HNF6 gene,
7) a combination of the HNF1 protein, the FOXA gene, and the HNF6 protein, and
8) a combination of the HNF1 protein, the FOXA protein, and the HNF6 protein.

In one embodiment, the combination as described herein can be a combination of the HNF1 gene, the FOXA gene, and the HNF6 gene, for example, a combination of HNF1 mRNA, FOXA mRNA, and HNF6 mRNA.

HNF1 (hepatocyte nuclear factor 1) is a protein having a homeodomain and has two types of isoforms, HNF1A and HNF1B.

HNF6 (hepatocyte nuclear factor 6) is a homeodomain transcriptional factor involved in histogenesis in humans and controls the expression of various liver genes while controlling the development of diverse tissues of the pancreas, the liver, and the like.

FOXA is a hepatocyte nuclear factor (transcriptional factor) necessary for the earliest stage of liver tissue formation and includes FOXA1, FOXA2, and FOXA3. These FOXA transcriptional factors are considered to have functional complementarity to each other because of having 90% or higher homology at the amino acid level in a common forkhead/winged helix domain.

The combination as described herein may comprise an additional factor. In the case of comprising an additional factor, preferably, the factor does not substantially reduce a disease-treating and/or - preventing effect. Examples of the factor that may be added to the combination include MYC gene or protein. MYC family genes are known as transcriptional factors that work by binding to nuclear DNA, and include c-MYC, L-MYC, and N-MYC in humans.

Table 1 shows the amino acid sequences of the reprogramming factors and the nucleotide sequences of genes encoding these factors.

**[Table 1]**

| Reprogramming factor | Nucleotide sequence | Amino acid sequence |
|---|---|---|
| HNF1A NM_000545 | SEQ ID NO: 1 | SEQ ID NO: 2 |
| HNF6 NM_004498 | SEQ ID NO: 3 | SEQ ID NO: 4 |
| FOXA1 NM_004496 | SEQ ID NO: 5 | SEQ ID NO: 6 |
| FOXA2 NM_021784 | SEQ ID NO: 7 | SEQ ID NO: 8 |
| FOXA3 NM_004497 | SEQ ID NO: 9 | SEQ ID NO: 10 |

The genes encoding these factors or their fragments can be cloned with reference to, for example, "Molecular Cloning, A Laboratory Manual (4th edition)" (Cold Spring Harbor Laboratory Press (2012)) or may be obtained from addgene and the like.

The gene as used herein encoding HNF1A is referred to as "HNF1A gene", and the gene encoding HNF6 is referred to as "HNF6 gene". Genes encoding other factors can also be expressed as described above.

The reprogramming factors (genes or proteins) as used herein are not limited by nucleic acid sequences having the nucleotide sequences represented by SEQ ID NOs shown in Table 1 or amino acid sequences having the amino acid sequences shown therein. The following mutant forms can also be used as long as the mutant forms have functions of the reprogramming factors:
(a) a protein that consists of an amino acid sequence derived from the amino acid sequence shown in Table 1 (amino acid sequence represented by SEQ ID NO: 2, 4, 6, 8, or 10) by the deletion, substitution, or addition of one or several (e.g., 10 or less, 5 or less, 4 or less, 3 or less, or 2) amino acids, and has functions as a reprogramming factor;
(b) a protein that consists of an amino acid sequence having 80% or higher, 85% or higher, 90% or higher, 95% or higher, 98% or higher, or 99% or higher (e.g., 90% or higher) sequence identity to the amino acid sequence shown in Table 1 (amino acid sequence represented by SEQ ID NO: 2, 4, 6, 8 or 10), and has functions as a reprogramming factor;
(c) a nucleic acid encoding the protein (a);
(d) a nucleic acid encoding the protein (b); and
(e) a nucleic acid that hybridizes under stringent conditions with a nucleic acid consisting of a nucleotide sequence complementary to the nucleotide sequence shown in Table 1 (amino acid sequence represented by SEQ ID NO: 1, 3, 5, 7 or 9), and encodes a protein having functions as a reprogramming factor.

The "stringent conditions" as used herein can be, for example, conditions of 5 × SSC, 5 × Denhardt's solution, 0.5% SDS, 50% formamide, and 50°C. Under these conditions, it can be expected that DNA and/or RNA having high homology can be obtained more efficiently with increase in temperature. However, possible elements that influence the stringency of hybridization are plurality of elements such as temperatures, DNA and/or RNA concentrations, DNA and/or RNA length, ionic strength, times, and salt concentrations. Those skilled in the art are capable of achieving similar stringency by appropriately selecting these elements.

Whether or not a specific protein has functions as a reprogramming factor can be examined by a well-known method. For example, the combination as described herein, comprising the specific protein, may be introduced to, for example, cells that are neither hepatic stem cells nor hepatic progenitor cells, which are then cultured for a predetermined period, and then the specific protein can be examined on the basis of whether or not the expression of hepatic stem cell or hepatic progenitor cell markers, for example, albumin, α-fetoprotein, and E-cadherin, is increased in the cells.

The combination as described herein can be, for example, but not limited to, any of the following combinations (1) to (3), for example, a combination (2) or (3):
(1) a combination of (a) the HNF1A gene or the HNF1A protein, (b) the FOXA3 gene or the FOXA3 protein, and (c) HNF6 gene or HNF6 protein,
(2) a combination of (a) the HNF1A gene, (b) the FOXA3 gene, and (c) HNF6 gene, and
(3) a combination of (a) the HNF1A protein, (b) the FOXA3 protein, and (c) HNF6 protein.

The combination may comprise these factors or may consist of or consist essentially of these factors.

The reprogramming factors can be produced in a genetic engineering manner (see, for example, "Molecular Cloning, A Laboratory Manual (4th edition)" (Cold Spring Harbor Laboratory Press (2012)).

When the reprogramming factors are genes, the genes may be, for example, DNA or RNA (e.g., mRNA). The reprogramming factors may be contained in vector(s). In one embodiment, the present invention relates to vector(s) comprising the combination as described herein. The vector(s) may be an expression vector(s) in which the reprogramming factors and/or an additional DNA sequence are operably linked to one or more expression control sequences. Examples of the expression control sequence include promoters, enhancers, and terminators. When a gene is linked to an expression control sequence and is capable of being expressed in cells or the like, this may be referred to as being "operably linked" as used herein. By operably linking each of the reprogramming factors to a hepatocyte-specific promoter in the vector, specificity for hepatocytes can be enhanced and effects can be improved and/or side effects can be reduced. Examples of the hepatocyte-specific promoter include thyroxine-binding globulin (TBG) gene promoter, albumin gene promoter, transthyretin (TTR) gene promoter, apolipoprotein gene promoter, and Pr1 promoter.

All the genes encoding the reprogramming factors may be contained in one vector or may be contained one by one in separate vectors. Alternatively, some of the genes may be contained in one vector, and the remaining genes can be contained in another vector.

Examples of the expression vector that may be used in the present specification include plasmids and bacteriophages as well as virus vectors derived from retrovirus, vaccinia virus, adenovirus, lentivirus, adeno-associated virus (e.g., AAV8), and Sendaivirus. Use of AAV8 having high affinity for hepatocytes can enhance specificity for hepatocytes and improve effects and/or reduce side effects.

In one embodiment, the combination described in the present specification is contained in lipid nanoparticles (LNP). In one embodiment, the present invention relates to LNP comprising the combination as described herein.

When the reprogramming factors are proteins, introduction efficiency into cells can be enhanced by linking the proteins to membrane-permeable peptides or the like or using cationic lipids.

The combination as described herein can be administered to a subject by injection, for example, to a skin tissue, into the vascular vessel (e.g., into the vein), or into the peritoneal cavity in an animal.

In one embodiment, the combination as described herein is contained in one or more pharmaceutical compositions.

The pharmaceutical composition as described herein can further contain, in addition to the combination described in the present specification, a pharmaceutically acceptable carrier (excipient, bulking agent, binder, lubricant, etc.) and/or a known additive (buffer, tonicity agent, chelating agent, colorant, preservative, fragrance, flavor, sweetener, etc.). For example, lipid nanoparticles (LNP) can be contained as the carrier to thereby promote gene delivery.

The pharmaceutical composition as described herein can usually be administered systemically or locally in an oral or parenteral form. The pharmaceutical composition as described herein can be administered to a living body by, for example, intravenous injection, intramuscular injection, intraperitoneal injection, subcutaneous injection, or a suppository, and can be administered by, for example, intravenous injection. The dose of the medicament as described herein can be appropriately set depending on age, a body weight, symptoms, a therapeutic effect, an administration method, a treatment time, and the like. For example, the pharmaceutical composition or an active ingredient can be administered in an amount of 0.0001 to 100 mg/kg, preferably 0.01 to 100 mg/kg, per day to an individual.

The pharmaceutical composition as described herein can be prepared as a pharmaceutically acceptable formulation. The formulation can be prepared as an injection such as an aseptic solution, a liquid suspension, or a freeze-dried formulation, or a tablet, granules, a powder, a capsule, an emulsion, a suspension, a syrup, or the like according to an approach usually performed.

The "subject" to which the combination or the pharmaceutical composition as described herein is administered includes a human or a non-human animal. Examples of the non-human animal include birds and non-human mammals (e.g., bovines, monkeys, cats, mice, rats, guinea pigs, hamsters, pigs, dogs, rabbits, sheep, and horses).

In one embodiment, the present invention relates to a method for treating and/or preventing liver disease, comprising administering the combination as described herein, or HNF1 gene or HNF1 protein, FOXA gene or FOXA protein, and HNF6 gene or HNF6 protein to a subject. The combination, the HNF1 gene or the HNF1 protein, the FOXA gene or the FOXA protein, the HNF6 gene or the HNF6 protein, and the liver disease according to the present embodiment are as described in the present specification.

### Examples

### <Example 1: Dedifferentiation of hepatocyte via in vivo reprogramming>

In this Example, a liver fibrosis model (liver cirrhosis model) was used which was prepared by mixing carbon tetrachloride (FUJIFILM Wako Pure Chemical Corp.) with olive oil (Nacalai Tesque, Inc.) and subcutaneously injecting the mixture at a dose of 5 µl per g body weight of a mouse to a C57BL/6J mouse (KBT Oriental Co., Ltd.) twice a week for 8 weeks. AAV8, which is adeno-associated virus (AAV) having high affinity for hepatocytes, was used for the introduction of human induced hepatic progenitor cell (human iHepPC) inducing factors (FOXA3, HNF1A, and HNF6: referred to as h3F). Specifically, a vector comprising each of the inducing factors was prepared by recombining a Cre sequence of AAV.TBG.PI.Cre.rBG (Plasmid #107787) (Addgene) with the sequence of each inducing factor (nucleotide sequence of FOXA3, HNF1A, or HNF6 shown in Table 1) according to a routine method. HEK293 cells were transfected with the resulting vectors using polyethyleneimine (PEI Max) (Polysciences, Inc.) according to the attached protocol to prepare AAV8. AAV8 was injected to the tail vein only once after a lapse of 4 weeks from the start of carbon tetrachloride administration. While the carbon tetrachloride administration was then continued, gene expression analysis by qPCR and protein expression analysis by immunostaining were conducted on α-fetoprotein (AFP) and epithelial cell adhesion molecule (EpCAM) using the liver 2, 4, 7, 14, and 28 days later from AAV8 administration. The gene expression analysis and the immunostaining were performed according to the methods described in Hiroki Inada et al., Nature Communications, Vol. 11, Article number: 5292, 2020. AFP is a hepatic progenitor cell marker, and EpCAM is expressed in hepatic progenitor cells and biliary epithelial cells. The human iHepPC-inducing factors are expressed mainly in hepatocytes because the expression of the human iHepPC-inducing factors is controlled by hepatocyte-specific thyroxine-binding globulin (TBG) gene promoter in AAV8.

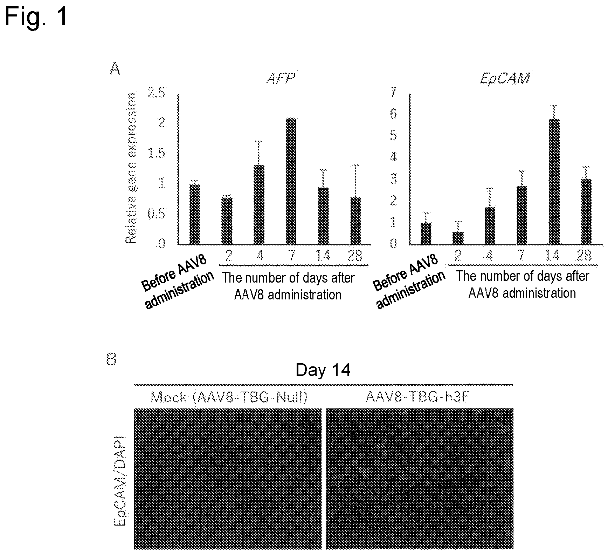

The results are shown in Figure 1. The gene expression levels of AFP and EpCAM were found to be elevated, peaking at 7 days and 14 days later, respectively, from the expression of the human iHepPC-inducing factors (A). The values of the graph depict relative values based on the gene expression levels in the mouse liver immediately before administration of AAV8 after 4-week administration of carbon tetrachloride.

Many EpCAM-positive cells were detected by immunostaining in the liver in which the human iHepPC-inducing factors were expressed, as compared with the liver of a mock control mouse given an empty vector (AAV8-TBG-Null) containing no human iHepPC-inducing factors (B). DNA of the cells was stained with DAPI (blue).

### <Example 2: Liver fibrosis improving effect brought about by dedifferentiation of hepatocyte via in vivo reprogramming>

In this Example, a liver cirrhosis mouse model prepared in the same manner as in Example 1 was used, and AAV8 containing the human iHepPC-inducing factors was introduced thereto after a lapse of 4 weeks from the start of carbon tetrachloride administration in the same manner as in Example 1. The carbon tetrachloride administration was then continued, and the analysis of liver tissue fibrosis and Col1a1 gene expression and the measurement of serum ALT and AST values were performed after a lapse of another 4 weeks. Specifically, after formalin fixation of a liver tissue, paraffin sections were prepared, and a fibrotic tissue area stained with Sirius Red was quantified using KEYENCE BZ-X800. cDNA was synthesized using Rever Tra qPCR RT kit (Toyobo Co., Ltd.) from mRNA extracted from the liver tissue using ISOGEN II (Nippon Gene Co., Ltd.), and the Col1a1 gene expression analysis was conducted by qPCR using the cDNA and using specific primers (TCCTCCAGGGATCCAACGA (SEQ ID NO: 11) and GGCAGGCGGGAGGTCTT (SEQ ID NO: 12)). The ALT value and the AST value in serum were measured using Transaminase CII-Test Wako (FUJIFILM Wako Pure Chemical Corp.). An empty vector (AAV8-TBG-Null) containing no human iHepPC-inducing factors was used as a mock control.

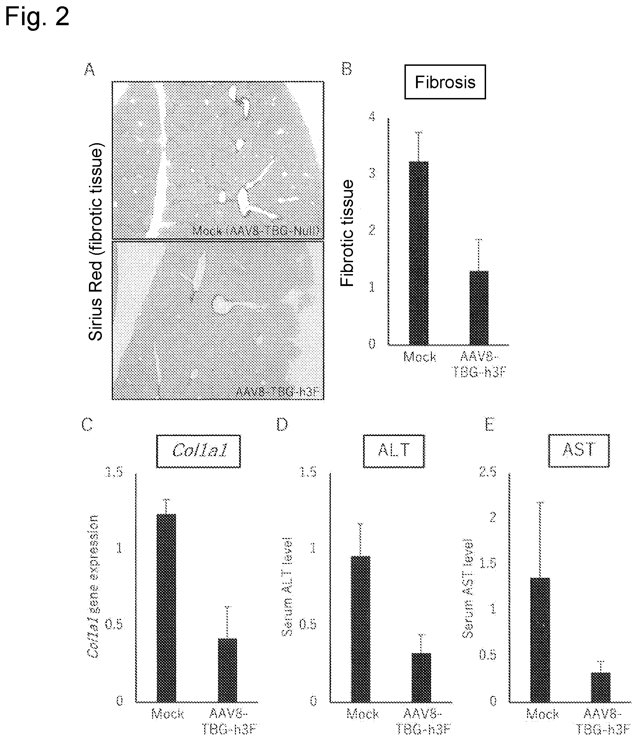

The results are shown in Figure 2. In the staining of the fibrotic tissue with Sirius Red, the amount of the fibrotic tissue stained red was decreased by the expression of the human iHepPC-inducing factors (A and B), and the gene expression level of collagen (Col1a1), a main component of the fibrotic tissue, was also decreased (C). The amounts of the liver damage markers alanine aminotransferase (ALT) and aspartate aminotransferase (AST) in serum were also decreased by the expression of the human iHepPC-inducing factors (D and E). The values of the graphs of Figures 2B to 2E each indicate a value obtained by dividing an endpoint value of week 8 by a value of week 4 immediately before administration of AAV8.

### <Example 3: Fatty liver improving effect brought about by dedifferentiation of hepatocyte via in vivo reprogramming>

In this Example, a liver cirrhosis mouse model prepared in the same manner as in Example 1 was used, and AAV8 containing the human iHepPC-inducing factors was introduced thereto after a lapse of 4 weeks from the start of carbon tetrachloride administration in the same manner as in Example 1. The carbon tetrachloride administration was then continued, and fat deposition in a liver tissue was measured after a lapse of another 4 weeks. Specifically, frozen sections of the liver tissue were prepared, and a fat deposition area stained with Oil Red O was quantified using KEYENCE BZ-X800. An empty vector (AAV8-TBG-Null) containing no human iHepPC-inducing factors was used as a mock control.

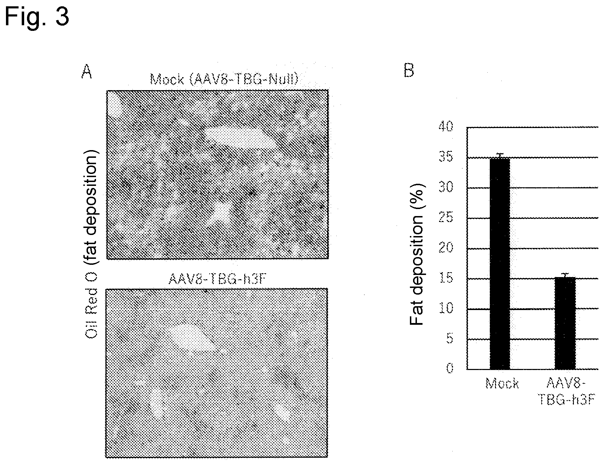

The results are shown in Figure 3. In the staining of fat droplets with Oil Red O, the area of a fat deposition region stained red was decreased by the expression of the human iHepPC-inducing factors (A and B).

### <Example 4: Liver histology improving effect brought about by dedifferentiation of hepatocyte via in vivo reprogramming>

In this Example, a liver cirrhosis mouse model prepared in the same manner as in Example 1 was used, and AAV8 containing the human iHepPC-inducing factors was introduced thereto after a lapse of 4 weeks from the start of carbon tetrachloride administration in the same manner as in Example 1. The carbon tetrachloride administration was then continued, and liver histology was analyzed after a lapse of another 4 weeks. Specifically, after formalin fixation of a liver tissue, paraffin sections were prepared and stained with hematoxylin-eosin (HE), and ballooning hepatocytes, steatotic hepatocytes, and the degree of inflammatory cell infiltration were then scored. A specific method for the scoring was performed as described in David E Kleiner et al., Hepatology, 2005 Jun, 41 (6): 1313-21. An empty vector (AAV8-TBG-Null) containing no human iHepPC-inducing factors was used as a mock control.

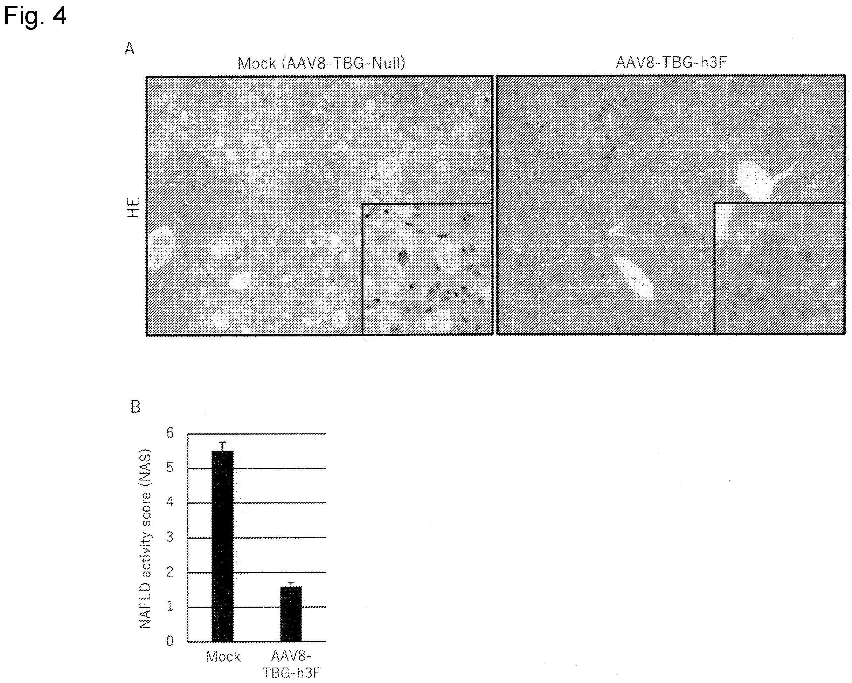

The results are shown in Figure 4. As a result of histopathological analysis by hematoxylin-eosin (HE) staining, ballooning hepatocytes, steatotic hepatocytes, and inflammatory cell infiltration were improved by the expression of the human iHepPC-inducing factors, and NAFLD activity score (NAS) was decreased (A and B).

### <Example 5: Liver fibrosis improving effect brought about by expression of human iHepPC-inducing factors using LNP>

In this Example, a liver cirrhosis mouse model prepared in the same manner as in Example 1 was used, and lipid nanoparticles (LNP) containing messenger RNA (mRNA) of human induced hepatic progenitor cell (human iHepPC) inducing factors (FOXA3, HNF1A, and HNF6: referred to as h3F) were administered at a dose of 2 µg mRNA /g (mouse body weight) once per five days a total of 6 times after a lapse of 4 weeks from the start of carbon tetrachloride administration. The preparation of mRNA-containing LNP was consigned to GenScript. The mRNA sequence of FOXA3 used in this Example is shown in SEQ ID NO: 13, the mRNA sequence of HNF1A is shown in SEQ ID NO: 14, and the mRNA sequence of HNF6 is shown in SEQ ID NO: 15. Carbon tetrachloride was also continuously administered during the period of LNP administration, and the analysis of liver tissue fibrosis and Col1a1 gene expression and the measurement of serum ALT and AST values were performed after a lapse of 4 weeks from the start of LNP administration in the same manner as in Example 2. LNP containing mRNA of green fluorescence protein (GFP) (LNP-GFP) was used as a control.

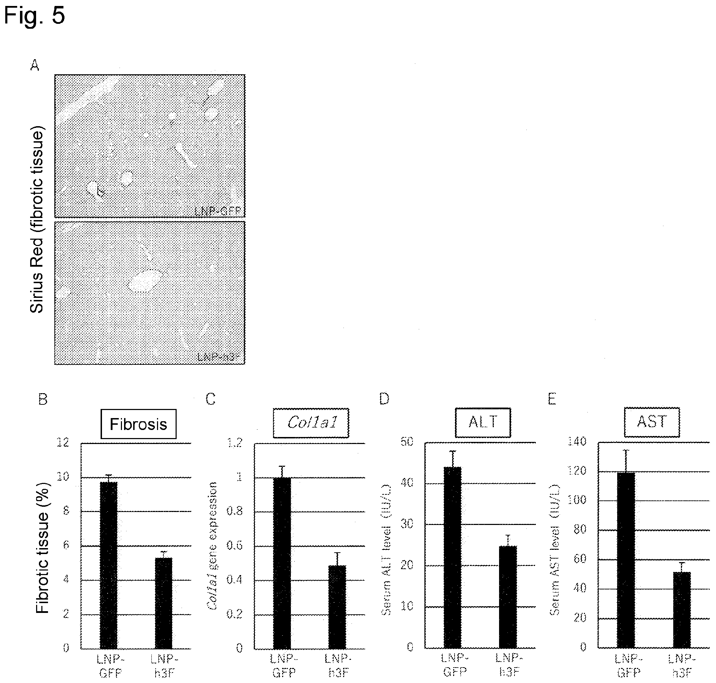

The results are shown in Figure 5. In the staining of the fibrotic tissue with Sirius Red, the amount of the fibrotic tissue stained red was decreased by the expression of the human iHepPC-inducing factors (A and B), and the gene expression level of collagen (Col1a1), a main component of the fibrotic tissue, was also decreased (C). The amounts of the liver damage markers alanine aminotransferase (ALT) and aspartate aminotransferase (AST) in serum were also decreased by the expression of the human iHepPC-inducing factors (D and E).

### <Example 6: Fatty liver improving effect brought about by expression of human iHepPC-inducing factors using LNP>

In this Example, a liver cirrhosis mouse model prepared in the same manner as in Example 1 was used, and lipid nanoparticles (LNP) containing messenger RNA (mRNA) of human induced hepatic progenitor cell (human iHepPC) inducing factors (FOXA3, HNF1A, and HNF6: referred to as h3F) were administered once per five days a total of 6 times after a lapse of 4 weeks from the start of carbon tetrachloride administration in the same manner as in Example 5. Carbon tetrachloride was also continuously administered during the period, and fat deposition in a liver tissue was measured after a lapse of 4 weeks from the start of LNP administration in the same manner as in Example 3. LNP containing mRNA of green fluorescence protein (GFP) (LNP-GFP) was used as a control.

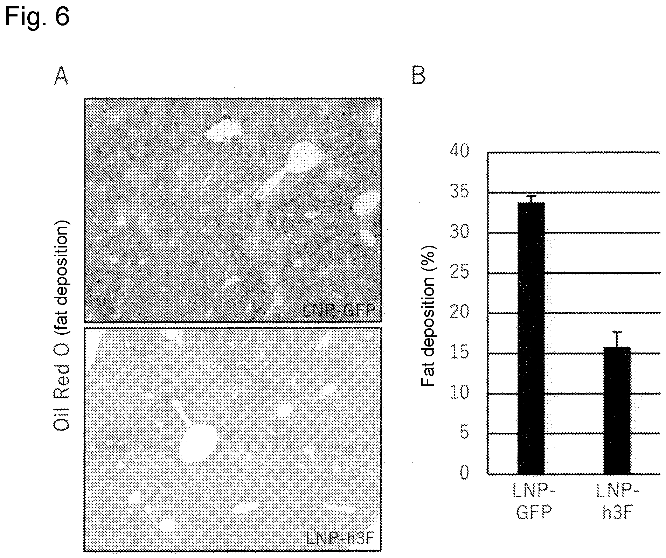

The results are shown in Figure 6. In the staining of fat droplets with Oil Red O, the area of a fat deposition region stained red was decreased by the expression of the human iHepPC-inducing factors (A and B).

### <Example 7: Liver histology improving effect brought about by expression of human iHepPC-inducing factors using LNP>

In this Example, a liver cirrhosis mouse model prepared in the same manner as in Example 1 was used, and lipid nanoparticles (LNP) containing messenger RNA (mRNA) of human induced hepatic progenitor cell (human iHepPC) inducing factors (FOXA3, HNF1A, and HNF6: referred to as h3F) were administered once per five days a total of 6 times after a lapse of 4 weeks from the start of carbon tetrachloride administration in the same manner as in Example 5. Carbon tetrachloride was also continuously administered during the period, and liver histology was analyzed after a lapse of 4 weeks from the start of LNP administration in the same manner as in Example 4. LNP containing mRNA of green fluorescence protein (GFP) (LNP-GFP) was used as a control.

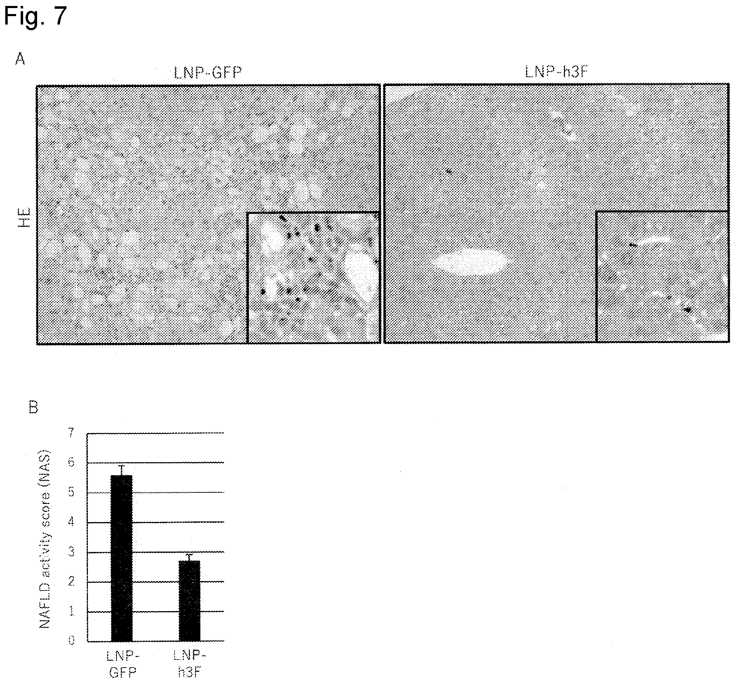

The results are shown in Figure 7. As a result of histopathological analysis by hematoxylin-eosin (HE) staining, ballooning hepatocytes, steatotic hepatocytes, and inflammatory cell infiltration were improved by the expression of the human iHepPC-inducing factors, and NAFLD activity score (NAS) was decreased (A and B).

## Claims

1. A combination for treating and/or preventing liver disease, the combination comprising HNF1 gene or HNF1 protein, FOXA gene or FOXA protein, and HNF6 gene or HNF6 protein.

2. The combination according to claim 1, wherein the liver disease is selected from the group consisting of liver cirrhosis, liver fibrosis, and nonalcoholic steatohepatitis (NASH).

3. The combination according to claim 1, wherein the liver disease is liver cirrhosis or liver fibrosis.

4. The combination according to claim 1, wherein HNF1 is HNF1A.

5. The combination according to claim 1, wherein FOXA is FOXA3.

6. The combination according to claim 1, wherein the combination comprises the HNF1 gene, the FOXA gene, and the HNF6 gene.

7. The combination according to claim 6, wherein the HNF1 gene, the FOXA gene, and the HNF6 gene are contained in a vector.

8. The combination according to claim 7, wherein the HNF1 gene, the FOXA gene, and the HNF6 gene are contained in separate vectors.

9. The combination according to claim 7, wherein the vector is adeno-associated virus (AAV).

10. The combination according to claim 9, wherein the vector is AAV8.

11. The combination according to claim 8, wherein the HNF1 gene, the FOXA gene, and the HNF6 gene are operably linked to hepatocyte-specific promoters in the vector.

12. The combination according to claim 1, wherein the HNF1 gene, the FOXA gene, and the HNF6 gene are contained in lipid nanoparticles.

13. The combination according to claim 1, wherein the combination is a pharmaceutical composition.
